# EUROPEAN PATENT APPLICATION

(11) **EP 2 854 059 A2**
(43) Date of publication of application: **01.04.2015**
(21) Application number: 14186075.9
(22) Date of filing: 24.09.2014
(51) Int. Cl.: G06F 19/26, G06F 19/28, G06F 19/22

(54) **Method for storage and communication of personal genomic or medical information**

(30) Priority: 27.09.2013 US 201361883632 P
(71) Applicant: Orbicule BVBA, 3001 Heverlee (BE)
(72) Inventor: Peter, Schols, 3001 Heverlee (BE)
(74) Representative: Laenen, Bart Roger Albert

(57) **Abstract**

The present disclosure provides methods and systems allowing for the easy and quick analysis, interpretation and communication of a personal genome sequence. The methods and systems include an understandable visualization of the complex genome data and facilitate the user's exploration as to the relevance of particular genome variations.

## Description

### FIELD OF THE INVENTION

The invention relates generally to methods and systems for personal genome data storage, management, communication and visualisation.

### BACKGROUND TO THE INVENTION

Ever since the deciphering of the Human Genome, completed in 2003, our knowledge about genetics has increased exponentially and novel scientific findings about genotype-phenotype correlations come out every day. Together with the strong tendency of reducing DNA sequencing costs, personal genomics, the sequencing and analysis of individual human genomes, has become a reality. This evolution provides novel opportunities and solutions for medicine and health care systems.

The human genome sequence is almost exactly the same (99.9%) in all people and has approximately 3x10⁹ base pairs of DNA. The remainder 0.1% accounts for millions of locations where DNA differences (variations) occur in the genome. Variations showing a genotype-phenotype correlation are important knowledge because they may impact our life. For instance, they may indicate a predisposition to develop a certain disease or predict outcome of a particular disease treatment. Whole genome sequencing provides raw data on all 3x10⁹ base pairs of DNA and requires an analysis of what that data means or how it can be utilized in various clinical applications.

As of 2010, a growing number of companies are marketing genetic testing kits directly to consumers in order to inform them about their genome variations. Some of these tests are sold directly to consumers on the Internet. Subsequent communication about the genetic results occurs frequently through web applications. These applications allow online access to computing systems that extract particular genetic variations out of the total sequence data and report in a technical manner as to their relevance.

Counsyl and Recombine (https:// www.counsyl.com, http://www.recombine.com) communicate about the genetic results through a web application and online and hard copy reports. The two companies report on 109 respectively 184 recessively inherited conditions accompanied by brief descriptions and technical reports. The communication of the information is focused on physicians and health care providers which are familiar with genetic variations, but is difficult to understand for the end user. No related mobile application is available giving access to the genomic information and no tools are provided for the users to personalize the content or to share certain information with others. Little attention goes to how test results of more than 100 features are organized and presented to individuals (only alphabetically ordered, use of jargon, lots of text and none or few graphical elements, ...)

23andme (https://www.23andme.com) is another personal genomics company offering a web application used for communicating genetic results. It consists of popular descriptions of 254 genetic features and associated technical reports. Although reporting is more focused on end users, a related mobile application that provides easy and rapid access to the genetic and other personal data is missing. Also, the web application does not allow sharing of personal genetic information with physicians or others outside the network or survey offered by the web application. No particular attention is given as to how test results of more than 200 features are organized and presented to individuals (only alphabetically ordered per category)

More recently, mobile applications have developed in this area.

MyGenome by Illumina (https://itunes.app!e.com/us/app/mygenome/id516405838?mt=8)
is a mobile application used for communicating genetic results. The application reports back on 251 genetic conditions, giving brief descriptions, clinical reports, and a genome map. The genome map shows the SNP's (Single nucleotide polymorphisms) and base sequences, refers to the associated genes, and maps the genes on the chromosomes. Compared to the web application of 23andme, communication is focused on physicians rather than on the patient himself. The focus is rather clinical and the genetic results are thus difficult to understand for the end user. No related web application is available. The user is offered no tools to personalize the content in this mobile application or to share certain information with its physician or any other person. Again, no particular attention goes to how test results of more than 200 conditions are organized and presented to individuals (only alphabetically ordered).

The existing tools for interpretation and communication of next generation sequencing (NGS)raw data remain uninviting, of limited utility and too high-level for general clinicians or consumers, who do not necessarily have an extensive background in genetics. Nowadays, whole genome sequencing data is still predominantly used in academics and only gradually gains interest in daily clinical practice. A number of companies develop tools for analysis, annotation and interpretation of these raw sequencing data. However, existing approaches remain high-level, solely focused on experienced geneticists, often use complex user interfaces, lack flexible and responsive filtering, use limited annotation, and only few of them offer a complete solution for storage, analysis, and communication of whole genome sequencing data. A complete and user-friendly solution to handle whole genome data that can be used by as well an end-user, a family doctor, as a medical geneticist is currently not available. This slows down the use and integration of genetic data into daily medical practice.

Clearly, the existing tools lack flexibility and the reporting is meant for a physician audience. The consumer, be it a generalist clinician or the patient himself, is facing a big challenge in interpreting the transmitted genome information.

As mentioned in Exploring Personal Genomics by Dudley & Karczewski (2013) on page 74: "We expect that there will be a need to create many novel types and implementations of visualizations for personal genomic information, as the awareness and availability of personal genomics continues to expand beyond its current domains."

### SUMMARY OF THE INVENTION

It is an objective of the present invention to remedy all or part of the disadvantages mentioned above. The present invention fulfils these objectives by providing methods and systems allowing for the easy and quick interpretation of a personal genome sequence. The methods and systems create an understandable visualisation of the complex genome data and facilitate the user's exploration as to the relevance of particular genome variations. The present invention overcomes shortcomings of the conventional art and may achieve other advantages not contemplated by the conventional software and services.

In general terms, the present invention provides a method and/or system for efficient storage and/or communication of personal genome sequence data and/or medical information, making the relevant personal genome sequence and/or relevant medical information accessible on a mobile device or web application in an easy and efficient way.

In one embodiment, the present invention provides a method and/or system for efficient storage and/or efficient communication of complex personal genome sequence and/or medical information from a user on a mobile device or web application, which method and/or system comprises the steps of:
- obtaining personal genome sequence data and/or medical data from the user,
- sorting and recording the personal genome sequencing data and/or medical data into easily accessible personal genome sequence data comprising two categories including a category harboring variant sequences and a category harboring non-variant sequences.

In one embodiment, the two categories include a priority category harboring variant sequences and a cold category harboring non-variant sequences.

In a related embodiment, the present invention provides a method and/or system for communicating complex personal genome sequence and/or medical information from a user on a mobile device or web application, which method and/or system comprises the steps of
- obtaining easy accessible personal genome sequence data and/or medical data from the user,
- providing one or more visual displays to the user based on the personal sequence and/or medical data,
- providing means in at least one of the visual displays to explore and navigate through the personal genetic or medical data.

In a specific embodiment, the present invention provides a method and/or system for communicating complex personal genome sequence and/or medical information from a user on a mobile device or web application, which method and/or system comprises the steps of
- obtaining personal genome sequence data and/or medical data from the user,
- sorting and recording the personal genome sequencing data into easily accessible personal genome sequence data comprising two categories including a priority category harboring variant sequences and a cold category harboring non-variant sequences,
- exploring the variants in the priority category for known variants,
- providing one or more visual displays to the user based on the explored personal sequence and/or medical data,
- providing means in at least one of the visual displays to explore and navigate through the personal genetic or medical results.

In certain embodiments, the method and/or system comprises the further step of:
- providing means in at least one of the visual displays for making personalized notes associated with the personal sequence and/or medical data.

In certain embodiment, the method and/or system comprises the further step of:
- providing means for unique integrated communication by voice or videoconference.

In certain embodiments, the method and/or system provides means to import all personal variant sequences and add annotation from external data sources to each variant.

In certain embodiments, the method and/or system further provides means for assigning scores to the personal variant sequences.

In certain embodiments, the method and/or system further provides means for parsing the natural language query and intelligent filtering and ranking of the variants.

In certain embodiments, the method and/or system further provides means for displaying a subset of variants and their annotated information, relevant for the specific query, as output.

In certain embodiments, the method and/or system further provides means for selecting specific variants, adding personal notes and additional scientific publications, and compiling a final report.

In one aspect, the invention provides for a method of medical information management by an application, which method comprises the steps of:
obtaining, with the application, medical information from the user;
sorting with the application the medical information into a two-layered storage format; and
recording the sorted medical information at a computer readable medium accessible by the application.

More particularly, the medical information comprises personal genome sequence information, and the two-layered storage format comprises at least two categories of sequences: a priority category harboring personal variant sequences and a cold category harboring personal non-variant sequences in a highly compressed format.

The method of medical information management further comprises:
facilitating exploration of the personal variant sequences in the priority category by the user through the application;
providing one or more visual displays to the user based on the personal variant sequence; and
providing means in at least one of the visual displays for a user to explore and navigate through the personal genome sequence information.

More particularly, the step of facilitating exploration of the personal variant sequences in the priority category comprises the step of filtering the personal variant sequences for genotype-feature correlations.

In particular, the step of facilitating exploration of the personal variant sequences in the priority category comprises the steps of:
identifying genotypes in the personal variant sequence;
correlating identified genotypes to colors to identify a relevance of an identified genotype; and
visually presenting the colors to convey the relevance of the identified genotypes.

More particularly, the step of correlating identified genotypes to colors in the personal variant sequence further comprises the steps of genotype-feature and genotype-feature-color correlation.

In particular, the step of facilitating exploration of the personal variant sequences in the priority category comprises the steps of:
identifying genotypes in the personal variant sequences;
correlating identified genotypes to a specific feature;
correlating a specific feature based on the identified genotype to colors to identify the relevance of a feature; and
visually presenting the colors to convey the relevance of the identified genotype.

In particular, the method of medical information management further comprises:
providing means in at least one of the visual displays for making personalized notes associated with the personal genome sequence information.

In particular, the method of medical information management further comprises:
providing means through the application for integrated communication by voice or videoconference.

In a related aspect, the invention provides for a method of personal genome sequence data management, the method comprising:
obtaining personal genome sequence data, the personal genome sequence data comprising a plurality of personal variant sequences and a plurality of non-variant sequences;
sorting the obtained personal genome sequence data into a first category of personal genome sequence data comprising the personal variant sequences and a second category of personal genome sequence data comprising the non-variant sequences;
storing the sorted variant sequences in a relational database optimized for fast access to the stored variant sequences;
storing the sorted non-variant sequences in a format configured to maximize compression.
filtering the personal variant sequences with a plurality of genotype-feature correlations to identify a plurality of genetic features;
linking each of the identified plurality of genetic features to a personal variant sequence of the plurality of personal variant sequences;
providing a visual presentation of the identified plurality of genetic features based upon the personal variant sequences;
providing a user interface to facilitate navigation of the personal genome sequence data presented in the visual presentation.

In particular, the step of providing a visual presentation comprises:
categorizing each of the identified genetic features based upon a relevance of the feature into a plurality of relevance categories, wherein a color code is indicative of the identified relevance category;
presenting the color code of the relevance category for each identified genetic feature in the visual presentation.

In particular, the step of providing a visual presentation further comprises:
presenting a graphical representation of a human body, the graphical representation of the human body comprising graphical representations of a plurality of body systems;
sorting the genetic features by an associated body system;
wherein upon receiving a selection of a body system, presenting the genetic features associated with the selected body system.

In particular, the step of providing a visual representation further comprises:
presenting a graphical representation of an organ within the graphical representation of a body system;
sorting the genetic features by an associated organ;
wherein upon receiving a selection of an organ within the graphical representation of a body system, presenting the genetic features associated with the selected organ.

In particular, the step of providing a visual representation further comprises:
presenting a graphical representation of at least one cell type within the graphical representation of the organ;
sorting the genetic features by an associated cell type;
wherein upon receiving a selection of a cell type within the graphical representation of the organ, presenting the genetic features associated with the selected cell type.

In particular, the step of providing a visual representation comprises:
presenting a graphical representation of human chromosomes; and
presenting an indication comprising the color code for each identified genetic feature in a location on at least one of the presented human chromosomes corresponding to a location on that chromosome of the personal variant sequence associated with the identified genetic feature.

In particular, the step of providing a visual representation comprises:
receiving a selection of a genetic feature from the a visual presentation;
generating a background information page for the selected genetic feature, the background information page comprising a presentation of at least one informational article regarding the selected genetic feature and a body system associated with the selected genetic feature, in conjunction with the identified relevance category, and the personal variant sequences associated with the selected genetic feature; and
visually presenting the generated background information page.

In a related aspect, the invention provides for a system for management of personal genome sequence data, the system comprising:
a computer processor operating on an application configured to obtain personal genome sequence data, the personal genome sequence data comprising a plurality of personal variant sequences and a plurality of non-variant sequences, the application sorts the personal genome sequence into a first category comprising the personal variant sequences and a second category comprising the non-variant sequence, and filters the personal variant sequences with a plurality of genotype-feature correlations to identify a plurality of genetic features linked to a personal variant sequence of the plurality;
a first computer readable medium configured to store the personal variant sequences in a relational database for fast access to the stored variant sequences;
a second computer readable medium configured to store the non-variant sequences in a format configured to maximize compression; and
a graphical user interface operating on a graphical display to visually present the plurality of identified genetic features based upon the personal variant sequences and to facilitate navigation of the personal variant sequences.

The invention can be implemented in numerous ways, including as a method; an apparatus; a system; a composition of matter; a computer program product embodied on a computer readable storage medium in a mobile device; a computer program linked to a web application; and/or a processor, such as a processor configured to execute instructions stored on and/or provided by a memory coupled to the processor. In this specification, these implementations, or any other form that the invention may take, may be referred to as techniques. In general, the order of the steps of disclosed processes may be altered within the scope of the invention.

Embodiments will be discussed with reference to the accompanying Fig.'s, which depict one or more exemplary embodiments. Embodiments may be embodied in many different forms and should not be construed as limited to the embodiments set forth herein, shown in the Fig.'s, and/or described below. Rather, these exemplary embodiments are provided to allow a complete disclosure that conveys the principles of the invention, as set forth in the claims, to those skilled in the art. For the purpose of clarity, technical material that is known in the technical fields related to the invention has not been described in detail so that the invention is not unnecessarily obscured.

### BRIEF DESCRIPTION OF THE DRAWINGS

The invention, both as to its structure and its operation, will be best understood from the accompanying drawings, taken in conjunction with the accompanying description, in which similar reference characters refer to similar parts, and in which:
Fig. 1 is an overview of a system to store and communicate personal genetic or medical information.
Fig. 2 is an exemplary representation of a navigation interface based on relevance.
Fig. 3 is an exemplary representation of a navigation interface based on body systems.
Fig. 4 is an exemplary representation of a navigation interface based on chromosomes.
Fig. 5 is an exemplary representation of a chromosome selection, zoomed-in.
Fig. 6 is an exemplary representation of a card interface following selection of a specific feature in one of the navigation interfaces.
Fig. 7 is an exemplary representation of an article interface following card navigation.

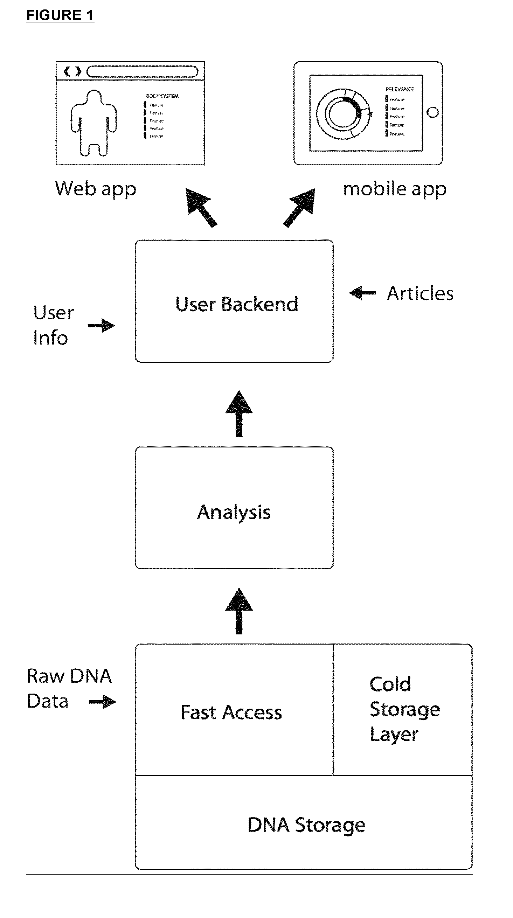

The interaction between the user and the visual display in figures 1 to 5 is explained by way of a device with a touch screen, on which the application is running, and one of the user's hands.

### DETAILED DESCRIPTION OF THE INVENTION

The present invention concerns a method of medical information management. The method is part of a complete service/product for consumers who may include physicians, including generalist physicians, or patients themselves, which includes the sequencing of an entire human genome, storage of this data, analysis, annotation and interpretation of important genetic variants and communication of these results in a clear way. It involves the novel use of mobile and web applications for storage, communication and visualization of personal medical test results and for providing users with necessary background information about biology and genetics in general and in their test results in particular.

An overview of the present method of medical information management by an application is depicted in Fig. 1. Raw sequencing data is first sorted and stored in an efficient way, providing a fast access to personal sequences for known common and rare variations in the human genome and a cold storage layer harboring personal sequences for genomic locations not susceptible to variation. During the analysis part, the personal variant sequences are assessed for known genotype-phenotype correlations. These analysis results are then securely sent to the User Backend, where also user info and content about the test features is stored. Finally, these data are combined in the mobile or web applications, which facilitate the user to explore and understand personal genomic information by means of several intuitive and graphic based user interfaces. As is disclosed by Fig. 1, embodiments as disclosed herein may operate in a "web app" implementation wherein the software is stored and executed at a server location apart from the user or the computing device operating as the user interface. In such implementations, the user's computing device may exemplarily be a mobile device, a tablet, laptop, or desktop computer, connected to the server where the software is executed by a communication network, exemplarily the internet.

The personal genome information or personal genome sequence information from a user contains information about the user's individual genes, variations associated to the genes and medical information associated to the known variation. The variations can be at the level of DNA or RNA. Genomic information can be generated from a body sample of the user using any of several methods well known in the art, such as, but not limited to, sequencing. As the user will need to submit a sample suitable for analysis, the method may require the steps of ordering for getting a sequence analysis and the step of providing a suitable sample for analysis. Once the sample is analysed, the results will be communicated. The resulting personal genome sequence data is mostly provided in a big file record encoded in a computer readable format. To store or access such big data files is often complex and as such is only used in certain research and clinical settings.

In order to give users, consumers and patients home setting access to their personal sequence information, a cloud-based storage and query system for genomic/medical data was developed. The system was designed in such a way that a) big raw data received from input devices and other sources are stored very efficiently, b) the relevant information is easily accessible, and c) the personal data is presented in formats that appeal to a broad audience. The present methods and systems encompass several ways to explore and navigate through the personal genetic and/or medical results and are focused on end users, patients and consumers themselves instead of highly trained medical staff. In order to achieve this, the pervasive usage of four colors: green, red, orange and gray; in combination with certain graphics, play a central role in the present system.

In a general aspect, the present invention provides a method and/or system for efficient storage and/or communication of personal genome sequence data and/or medical information, making the relevant personal genome sequence and/or relevant medical information accessible on a mobile device or web application in an easy and efficient way.

Thus, the present methods and/or systems apply to mobile devices or mobile applications or web applications. Mobile devices have been designed for many applications and include mobile computers, smartphones, and tablet computers. Web applications are applications in which all or some parts of the software are downloaded from the Web each time it is run.

With "method and/or system" is meant a method of medical information management. The personal genome sequence and/or medical information is meant to comprise the complete DNA sequence of a human genome (from the user). This comprises the diploid genotype (the order of DNA nucleotides, referred to as A, C, G and T) for about 98-99% of the 3x10⁹ base pairs of DNA (nuclear and mitochondrial human DNA). It provides raw data on nearly all 3x10⁹ base pairs that make up an individual's DNA. In certain embodiments, the personal genome sequence and/or medical information comprise only parts of the complete DNA sequence of a human genome.
Medical information may comprise proteomic, transcriptomic, metagenomic, epigenomic, exomic ... data, results from blood and lab tests, current treatments and drug regimens, ....

### Two-layered genome storage system

The present medical or genome information storage system is a cloud-based storage and allows for the handling of genome data in formats provided by all major suppliers, including, but not limited to, Illumina, BGI, Complete Genomics, etc. Most often, the raw personal genome sequence information obtained from sequencing providers, health care providers and other sources is available in text format.

In one embodiment, the methods and/or systems of the present invention include a step of sorting and recording the personal genome sequencing data into two categories (two-layered storage format). The system hereto scans the raw personal genome data for variations against one or more reference genome(s) and sorts and records the data into two priority layers or categories: a first layer or category that provides fast access to most commonly used variation identifiers, and a cold storage or category that maximizes compression for genomic locations that are not susceptible to variation (non-variant identifiers) and don't differ from the reference genome. The variations in the first layer include personal real and neutral variations. A neutral variation is a variation that does not differ from the reference genome but does position at a variant identifier location.

With "personal variant sequences" is meant variations in the first layer of the two-layered storage. Variations may amongst others include mutations, deletions, insertions, genetic rearrangements, polymorphisms, single-nucleotide polymorphisms (SNP's) and/or copy number variations. All personal variations relative to the reference genome are stored in a relational database system, allowing fast, real-time access for variants. Regions that are not susceptible to variation and that do not differ from the reference genome(s) are stored (recorded) in a highly compressed format, on long term disk storage (e.g. Amazon S3 / Amazon Glacier), maximizing the number of genomes that can be stored within a given space. This kind of storage has longer access times, as the genome has to be decompressed. However, since these regions do not differ from the reference genome, they will be accessed very rarely. The main reason for their storage, is to allow researchers and end users to assess the sequencing quality of individual bases (e.g. as a negative control). Additionally, unidentified variants may arise over time. Consequently, current sequence positions and regions occupied by non-variable sequences stored in the cold category may become identifiers for variation (e.g. low density variation) in later reporting and may require a track back as to their sequence or quality. As such, the cold storage leads to flexibility and sequence accessibility convenience for later stage analysis.

### Genomic analysis system:

Filtering the personal variant sequences for genotype-feature correlation facilitates exploration of the personal variant sequences in the priority category harboring personal variant sequences. The method and/or system will filter the personal variant sequences towards published or known genotype-feature correlations and link the public data to the associated personal variant. Based on the genotype-feature correlation, the personal variant is thus linked to for instance phenotypic features. For instance, the deltaF508 mutation in the CFTR gene, associated with cystic fibrosis, will categorise in the cystic fibrosis feature. Further links to non-public information may be joined to the personal variant sequences, including medical data and non-public research information. Thus, through filtering and linking steps, a relational database is created in the form of relational joined information gathered from different sources. All or part of the data and data-related information may be used for linkage. Useful sources comprise for instance papers from public databases, information from the Human Gene Mutation Database (HGMD), .... Medical information used for linkage may comprise proteomic, transcriptomic, metagenomic, epigenomic, microbiomic, medical, exomic ... data, which data may be public and/or personal data.

As used herein, "A reference genome", also known as a reference assembly, is a digital nucleic acid sequence database, assembled as a representative example of a species' set of genes. These reference genomes are often assembled from the sequencing of DNA from a number of individuals and provide a haploid mosaic of different DNA sequences from each individual. For instance, the Genome Reference Consortium human genome (build 37) or GRCh37 is derived from thirteen anonymous volunteers from Buffalo, New York.

As used herein, a 'feature' refers to a certain observable characteristic or trait (phenotype) of an organism, such as morphological, developmental, biochemical, physiological, conditional or behavioural properties. Feature and phenotype are used interchangeably throughout the text.

As used herein, a "test result" refers to personal genome sequence data (genotype) and/or medical data all or not linked to a feature.

In one embodiment, the methods and/or systems of the present invention include a step of data transmission requiring encrypted communication and secure identification. The data is stored very securely and special authorization procedures are integrated to allow access of the data. The data is stored anonymously on the platforms of a hosting provider such as Amazon AWS. Encrypted communication and secure identification of a network web server may happen through, for instance, HTTPS, using secure 256-bit SSL certificates. In one embodiment, the methods and/or systems of the present invention may require one or more identifiers such as a password in order to limit access by unauthorized users.

Based on the personal sequence and/or medical data, one or more visual displays are provided. In particular, information from the first layer record is used for the visual display. Each of the visual displays allows for navigation to explore the genetic and/or medical test results. In order to provide suitable visual displays, the methods and/or systems of the present invention include a step of linking the features to one or more graphical elements familiar to most people.

### Navigation based on personal relevance - Relevance interface

By using elements, such as color codes and a trackwheel, the method and/or system help the user to better understand its own genetic and medical test results. Users are able to explore their genetic or medical test results based on personal relevance.

In one embodiment, the methods and/or systems of the present invention include a step of linking the features to one or more color codes. Graphical elements that include a color code and a track-wheel are designed to organize the test results of the complete set of features in a logical way, in this case personal relevance, and as a guide for users to navigate. Any color code can be used. In preferred embodiments the color code comprises or consists of four colors. In preferred embodiments, the color code consists of green to indicate a result which is better than average; grey to indicate an average result; orange to indicate results that need attention; and red to indicate results that need immediate attention. It is shown as a small colored bar next to each tested feature. The color code is used throughout the whole application; each test has a particular color, based on a positive, neutral, negative or even more negative personal test result (e.g. in case of an autosomal recessive condition such as cystic fibrosis, being affected (two recessive CFTR alleles) will be indicated in red, being a carrier (one recessive CFTR allele) will be indicated in orange and being unaffected (no recessive CFTR alleles) will be indicated in grey). As a consequence, these colors will differ per test among individuals.

In one embodiment, the visual display depicts a trackwheel including the four colors. In this particular case, the trackwheel provides the means for exploring and navigating through the features associated with color code. With the aid of the trackwheel, the user can 'scroll' through the list of features tested, organized by personal relevance. The combination of the trackwheel and color code allows users to have a good overview of the complete set of features, organized by relevance of their own personal results. At one glance, the proportion of different types of results for a complete set of features is evident. From this point, users can explore test results and background information related to a particular feature in more detail by selecting the feature itself.

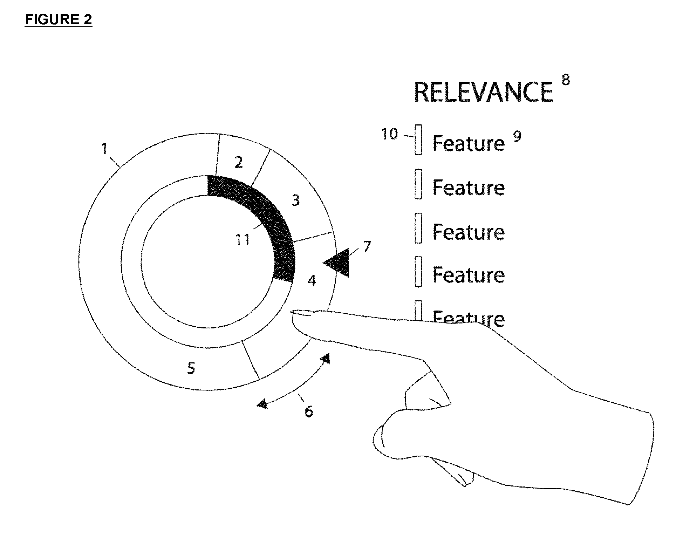

Fig. 2 is an exemplary representation of a navigation interface based on relevance, depicting a circular chart **1.** The chart is subdivided into four areas **2, 3, 4, 5.** Each area represents one of the four colors (green, grey, orange, red) used throughout the application. Each color corresponds to a certain relevance that a subtest can have for the user: positive, neutral, negative or even more negative.

When the user touches part of the chart and drags his finger, the touched part moves along **6** until the finger is lifted.

A triangle **7** indicates the selected area of the chart. The selection can be adjusted by the methods outlined in **6.** The name is displayed of the relevance **8** corresponding to the currently selected chart area **7.**

A list of tested features **9** is displayed that have a relevance corresponding to the currently selected chart area.

Each feature is accompanied by a colored bar **10.** The bar color corresponds to the relevance of the feature for the user, as used throughout the application. A second smaller circular chart **11** depicts a progress bar to indicate the amount of features viewed by the user.

### Navigation based on body system - body system interface

By using elements such as the representation of a human body and color codes, the method and/or system helps the user to better understand its own genetic and medical test results. Graphical elements that include a human body depicting the body systems are designed to organize the test results of the complete set of features or subtests in a logical way, in this case according to the body system, and as a guide for users to navigate.

In one embodiment, the methods and/or systems of the present invention include a step of linking the features to one or more body systems. The mass of genome data is given a meaning through the linking of the transmitted personal sequence to the body systems. The body depicting one or more body systems is used for easy interpretation of the transmitted genome information. Thus, the method provides record lists based on assignments of the features to a body system. One or more visual display outputs based on these record lists are provided to the user.

In one embodiment, the visual display depicts a body system in a body, preferably a human body, including the nervous, immune, lymphatic, endocrine, digestive, integumentary, urinary, reproductive, skeletal, muscular, cardiovascular, sensory, and respiratory systems. In this particular case, the body system provides the means for exploring and navigating through the personal genetic or medical results. Users can switch from one type of body system to another by tapping on or selecting the particular body system of interest in the body drawing.

The term "body system", also referred to as an organ system or biological systems, is a group of organs that work together to perform a certain task. The "cardiovascular system" is the body's transport system. It is made up of a group of organs that transport blood throughout the body. The "digestive system" is made up of organs that break down food into protein, vitamins, minerals, carbohydrates, and fats, which the body needs for energy, growth, and repair. The "integumentary system" includes skin, hair, fat and nails. The "endocrine system" is made up of a group of glands that produce the body's long-distance messengers, or hormones. The "immune system" is our body's defense system against infections and diseases. It filters out organisms that cause disease, produces white blood cells, and generates disease-fighting antibodies. The "lymphatic system" supports the immune system. The "muscular system" is made up of tissues that work with the skeletal system to control movement of the body. The "nervous system" is made up of the brain, the spinal cord, and nerves. The "reproductive system" allows humans to produce children. The "respiratory system" brings air into the body and removes carbon dioxide. It includes the nose, trachea, and lungs. The "skeletal system" is made up of bones, ligaments and tendons. It shapes the body and protects organs. The "urinary system" eliminates waste from the body, in the form of urine. The kidneys remove waste from the blood.The "sensory system" is responsible for vision, hearing, taste, touch and smell.

Linkage of organ systems (including particular organ, structure or cell type) and features is based on the main characteristic signs and symptoms of that feature - e.g. the feature cystic fibrosis is linked to the respiratory, digestive and reproductive systems since it causes the production of thick, viscous mucus in the lungs, hampering respiratory function, in the pancreas, hampering its production of digestive juices, and fertility problems due to thickened mucus at the cervix in females or the absence of a vas deferens in males, respectively.

Upon selecting the particular body system in the depicted body, a list of features related to that particular body system shows up. For instance, selecting the digestive system will show a list of features related to the digestive system and will include for instance the colorectal cancer feature amongst others.

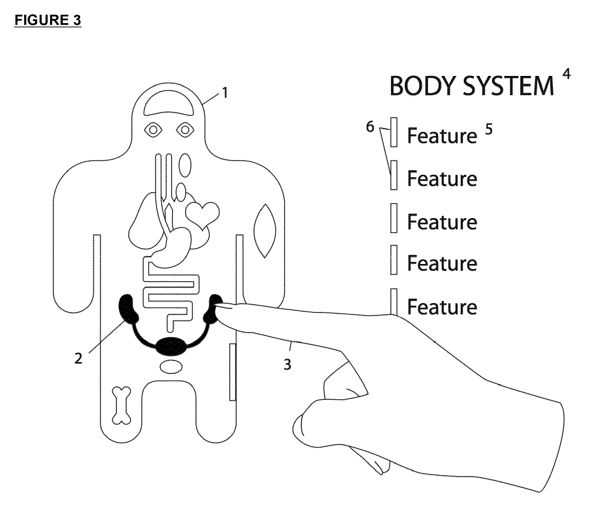

The method and/or system may display different levels of detail in connection to the depicted body system (Figure 3), including particular organ, structure or cell types. In such case, a list of relevant features associated with that particular organ, structure or cell type will be shown at the same time. For instance, the digestive system in the human body may be visualized by the digestive tract and a list of associated features. Further visual display may show a particular organ, structure or cell type in more detail. For instance, further levels of detail of the digestive system may depict the mouth, esophagus, stomach, small intestine, large intestine, rectum, anus and lists of associated features. Still further levels of detail may depict the salivary glands (in the mouth), digestive glands (in the stomach), or cell types associated to the glands and their respective lists of associated tests.

In one embodiment, graphical elements that include a representation of a human body depicting one or more body systems is used in combination with a color code designed to organize the test results of a complete set of features in a logical way.

In the navigation views of the application, small colored drawings (e.g. bars) will appear together with each feature in the list. The combination of the human body drawing and color code allows users to have a good overview of the complete set of features, organized by body system and at the same time, users have immediate information about the relevance of their own personal results per feature. From this point, users can explore test results and background information related to a particular feature in more detail by selecting the feature itself.

Fig. 3 is an exemplary representation of a navigation interface based on body systems, depicting the outline of a body in schematic form. Various body systems are overlaid **1.** The body systems have an opaque appearance to indicate that they can be selected, or a semi-transparent, dimmed appearance to indicate that they are not applicable in the current context. One body system is selected at the time and becomes highlighted upon selection. When the user interfaces **3** with or sufficiently close to the depicted urinary system **2,** the name of the selected body system is displayed **4** together with a listing of different features **5** related to the concerned body system **4.** The relevance of the feature for the user is emphasized by the colored bar next to the feature, as used throughout the application, attracting the attention to the user.

Furthermore, the human body may provide the possibility to filter on colors to have an overview of all features of a certain relevance and to which organ systems they belong.

### Navigation based on chromosomes - chromosome interface

In one embodiment, the methods and/or systems of the present invention include a step of linking the features to one or more chromosomes. Graphical elements that include a color code and the complete set of chromosomes are designed to organize the test results of the complete set of features in a logical way, in this case chromosome assignment, and as a guide for users to navigate. Individuals are able to explore their genetic or medical test results based on the location of the respective genetic marker on a human chromosome map. Graphical elements such as a map of the human chromosomes in combination with the color code are designed to organize the test results of the complete set of features in a logical way, in this case related to the genomic location of the genetic marker associated with the feature. Small colored (according to the color code) drawings (e.g. bars) on the chromosomes are used to depict a specific feature. The combination of the chromosome map and color code allows users to have a good overview of the complete set of features, organized by genomic location and at the same time, users have immediate information about the relevance of their own personal results per feature. From this point, users can explore test results and background information related to a particular feature in more detail by selecting the feature itself.

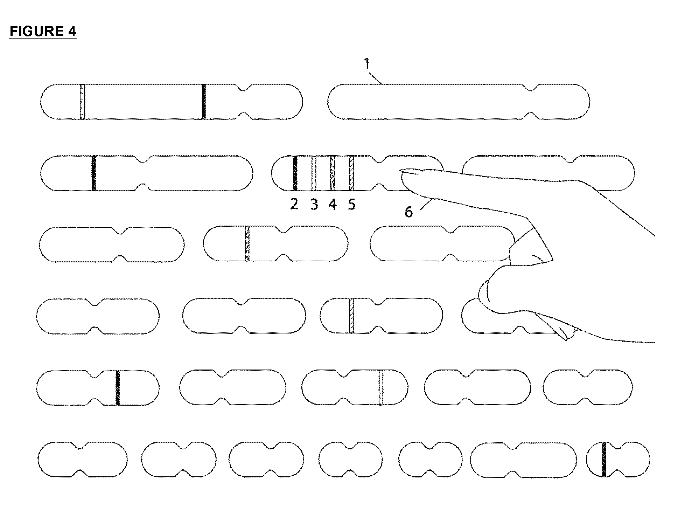

Fig. 4 is an exemplary representation of a navigation interface depicting the outline of a chromosome in schematic form. It describes the steps detailing the interaction between the user and the human chromosome map interface (here explained but not restricted to a device with a touch screen, on which the application is running, and one of the user's hands).

The navigation interface depicts a human chromosome map with all 22 autosomes (1-22) and the two sex chromosomes (XX in case of female individuals or XY, as in the present figure, in case of male individuals). Each chromosome is represented according to its size. Each chromosome contains small colored bars **2,3,4,5** representing features according to the genomic location of the genetic marker associated with the feature. Each color corresponds to a certain relevance that a test can have for the user: positive, neutral, negative or even more negative. Users can tap on or sufficiently close to a chromosome **6,** to select that particular chromosome **6.** Exactly one chromosome is selected at a time.

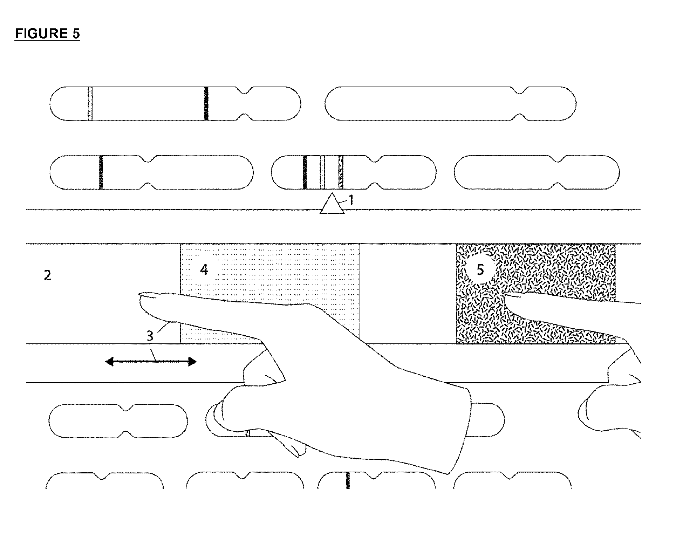

Figure 5 is a representation of a chromosome selection, zoomed-in.

Upon selection, an enlarged representation of that chromosome appears, in this case below **2,** at the same time, the other chromosomes lying above or underneath will move to the top and/or the bottom (up and/or down), in this way the zoom-in chromosome can appear in between. At the same time, the other chromosomes become more transparent, while the selected chromosome (both the miniature as the zoom-in) remains highlighted.

A triangular slider indicates the current position on the selected chromosome **1.** When the user touches the slider and drags his finger, the position on the selected chromosome changes until the finger is lifted, both in the small as the enlarged representation of the selected chromosome.

The enlarged representation of the selected chromosome is shown in **2.** Users can as well swipe through the enlarged representation of the selected chromosome, to move through the different locations **3.** The colored bars reveal more info in the enlarged representation of the selected chromosome **4,** including but not restricted to: the feature, the gene or SNP associated with that feature, the chromosomal location, etc.

Users can tap on a colored bar to explore test results and background information related to that particular feature **5.**

### Navigation based on categories

In one embodiment, the methods and/or systems of the present invention include a step of linking the features to one or more categories. Individuals will be able to explore their genetic or medical test results based on a set of categories. These categories will include: recessively inherited conditions, dominantly inherited conditions, drug response, health risk, lifestyle, etc.... Graphic icons are designed to represent each category. Users can tap/select one of these icons and upon this action a list of all features related to that particular category will appear. As in all navigation views of the application, the small colored bars will appear in front of each feature in the list. The combination of category icons and color code allows users to have a good overview of the complete set of features, organized by category and at the same time, users have immediate information about the relevance of their own personal results per feature. From this point, users can explore test results and background information related to a particular feature in more detail by selecting the feature itself.

The current prescribed graphics may be extended. Other types of nucleic acids, such as but not restricted to mitochondrial DNA, miRNA's, mRNA, tRNA, ... may be visualized on human chromosome maps.

### Cards interface

Users can explore test results and background information related to a particular feature in more detail by selecting the feature. Upon doing so, the navigation and communication elements of the present invention give access to a line-up of cards. Users can swipe to go through the cards. Each card represents a feature and will display a brief background summary, the results, and actionable feedback information in connection to the feature.

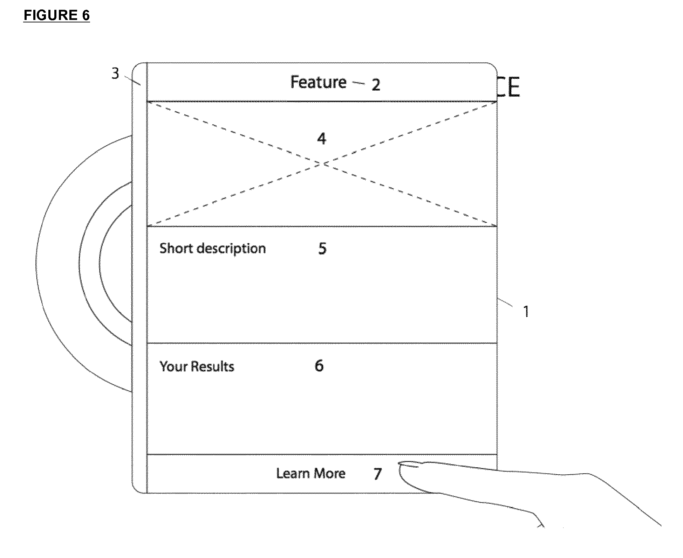

Figure 6 is an exemplary representation of a 'cards' interface. Each feature is represented by a card **1.** The top of the card displays the name of the feature **2.** The side of the card is colored in either grey, green, orange or red, and the applied color corresponds to the relevance of that feature for the user **3.** The applied color is used throughout the whole application. A drawing is depicted just underneath the name of the feature, illustrating that particular feature **4.** A short description of the feature is displayed **5,** as well as a brief explanation of the results **6.** Depending on the specific result, actionable feedback is given as a guide to treat or reduce the risk of developing the feature. This may include a numeration of constructive strategies and adequate safeguards the user can adopt in connection to the result. Users can tap on 'Learn more' at the bottom of the card to navigate further to the 'article' interface **7.**

### Article interface

The navigation and communication elements of the present invention allow for the election of features, giving access to a written article about a feature. The article contains detailed information organized in several paragraphs, such as introduction, symptoms, diagnosis, management, cause, inheritance, ..., and illustrations.

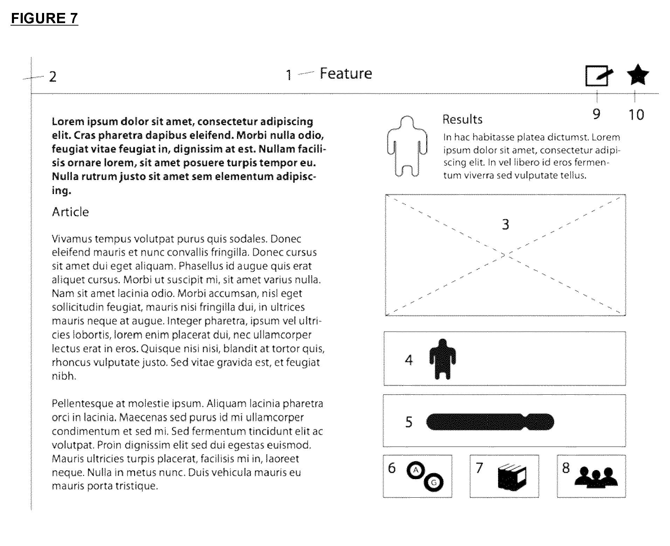

Figure 7 is an exemplary representation of an 'article' interface. The name of the feature is displayed at the top of the article **1.** The side of the article is colored in either grey, green, orange or red. The color corresponds to the relevance of that feature for the user, and is used throughout the entire application **2.** The article may contain specific illustrations such as still images or animations **3.** The article contains an interactive infographic of the human body, highlighting the specific body systems associated with that particular feature **4.** The article contains an interactive chromosome map, highlighting the location(s) of the genetic marker(s) associated with that particular feature **5.** The article contains a list of the SNP's tested for that feature **6.** A pop-up screen will appear upon tapping the icon. The article further contains literature information about the references used **7.** A pop-up screen will appear upon tapping the icon. The article contains a list of patient communities associated with that feature, organized by country **8.** A pop-up screen will appear upon tapping the icon. The user can make personalized notes per feature **9.** A pop-up screen will appear upon tapping the icon). These notes will be synced across applications (either mobile or web apps) and can easily be collected for discussion with a doctor or pharmacist. The user can label articles as favorite by tapping the star icon **10.** A list of personal favorites will be created following this action.

In one embodiment, the methods/systems as disclosed herein combine

Natural Language Query feature as already described above with the additional capability of natural language queries of the patient's DNA information, and particularly, the personal variant sequences.

In such an embodiment, the genomic analysis system is developed as a user-friendly system with a user interface that accepts natural language queries, allowing its use by either laymen or experienced geneticists. Such user interface may include a text field to receive natural language queries which may be incorporated into any of the user interfaces as disclosed herein.

Examples of queries may be "Show all pathogenic variants related to heart diseases", "What's this patient's cytalopram response?", or "Show all candidate variants causing mental retardation that are not present in father or mother". Subsequently, the genomic analysis system facilitates powerful and intelligent analysis, filtering, and ranking of the personal variant sequences to give as its output a subset of variants relevant for the query. In embodiments, features as disclosed herein are carried or in systems or methods to provide natural language queries.

In embodiments of the system and/or method, all personal variant sequences may be imported and extensive annotation added from external data sources to each variant. This may exemplarily be carried out at the user back end as described above. Annotated information may be regularly updated and may include but is not limited to: gene and protein information (NCBI, UniProt, etc.), frequency data (1000g, ESP, MAF, etc.), associated genetic conditions (HGMD, OMIM, etc.), associated pharmacogenomic data (PharmKgb, etc.), clinical significance data (dbSNP, clinvar, etc.), phenotypic traits and symptoms, associated inheritance pattern(s), its presence or absence in other family members, zygosity states (homozygous, heterozygous, hemizygous or compound heterozygous), functional and conservation scores (SIFT, Polyphen2, MutationTaster, PhyloP, GERP, etc.), functional element in the human genome (upstream, downstream, intragenic, coding region, splice region, transcription binding site, promotor region, etc.), effect (synonymous, missense, nonsense, frameshift, stop lost, start lost, etc.), expression data, gene and protein function, cellular pathways and network information (Reactome, UniProt, etc.), scientific publications (Pubmed, etc.).

Embodiments of the system and/or method further assign scores to the personal variant sequences. This may further be carried out in the analysis as described above exemplarily with respect to Fig. 1. The score of each variant is computationally calculated and based on the plurality of annotated inputs, e.g. a variant with a severe protein effect will receive a higher score compared to a variant with a neutral protein effect, a variant with a previously reported pathogenic clinical significance will receive a higher score compared to a variant of unknown clinical significance, a variant for which functional and conservation prediction scores exceed the threshold will receive a higher score compared to variants for which the scores remain below the threshold. In a further embodiment, this analysis is similar to that as described above with respect to color coding particular identified features in the individual, applied to personal variant sequences.

Embodiments of the system and/or method further receive a user input of a natural language query and then parse the natural language query to identify the key terms of the query. These terms are then used to filter the personal variant sequences. Based on the parsing, relevant scores for each variant will be taken into account in a filtering algorithm to filter the variants based on the query. Variants with high overall scores represent the most relevant variants with respect to the initial query and will appear on top of the list, variants with overall low scores represent less relevant variants with respect to the initial query. Embodiments may further rank the filtered variants based upon the previous assigned scores representing the severity of the personal variant sequence.

A subset of variants and their annotated information, relevant for the specific query, is displayed exemplarily on a graphical display, as an output. In the display the subset of variants may be ranked based on relevance and labeled with a color code (red-orange-green-grey) according to their relevance as described above.

Still further embodiments of the method and/or system enable the selection of specific variants, adding personal notes, and additional scientific publications, and compiling a final report. Apart from navigation and communication elements as disclosed above, the application supports integrated communication by voice and/or videoconference between the user and medical staff. This allows offering personal counseling about specific medical test results in a very convenient, user-friendly way. During voice or videoconference, medical staff can remotely take over the navigation of the user's mobile application (e.g. iPad) in order to lead the user through his medical test results. In addition, users can use the application in a very personal way, including but not restricted to a tool that allows making personal notes related to a specific feature.

The application allows users to make notes per feature and through a separate user interface all notes are collected and organized in a logical way, including but not restricted to: per feature, per use (family, physician, pharmacist, ...), etc. During a voice and/or videoconference, the user can then easily access his/ her specific notes.

Accordingly, in further one embodiment, the method comprises the further step of:
- providing means in at least one of the visual displays for making personalized notes associated with the personal sequence and/or medical data.

Further, in one embodiment, the method comprises the further step of:
- providing means for unique integrated communication by voice or videoconference.

The method and/or system of the present invention is made available as a Web App or a Mobile App. For implementation on the mobile device the method and/or system is available to the user in the form of an application (App) and can be downloaded to the mobile device via an application (App) store, website, ... online channels which are processes well known in the art and to which customers are familiar with. A mobile device leads to flexibility in working giving the power and convenience of quick internet and information access.

## Claims

1. A method of medical information management by an application operating on a computer processor, which method comprises the steps of:
obtaining, with the application, medical information from the user;
sorting with the application the medical information into a two-layered storage format; and recording the sorted medical information at a computer readable medium accessible by the application.

2. The method of claim 1, wherein the medical information comprises personal genome sequence information and wherein the two-layered storage format comprises at least two categories of sequences including a priority category harboring personal variant sequences and a category harboring personal non-variant sequences in a highly compressed format.

3. A method according to claim 2 further comprising:
facilitating exploration of the personal variant sequences in the priority category by the user through the application;
providing one or more visual displays to the user based on the explored personal variant sequence; and
providing means in at least one of the visual displays for a user to explore and navigate through the personal genome sequence information.

4. A method according to claim 3 in which the step of facilitating exploration of the personal variant sequences in the priority category comprises the steps of:
identifying genotypes in the personal variant sequence;
correlating identified genotypes to colors to identify a relevance of an identified genotype;
visually presenting the colors to convey the relevance of the identified genotypes; and
providing means in at least one of the visual displays for making personalized notes associated with the personal genome sequence information.

5. A method according to claim 3, further comprising:
providing means through the application for integrated communication by voice or videoconference.

6. A method according to claim 3 in which providing means for exploration and navigation comprises providing a colored trackwheel, in which colors on the colored trackwheel indicate the personal relevance of each feature identified by a personal variant sequence.

7. A method according to claim 3 in which the visual display includes a representation of a body depicting body systems.

8. A method according to claim 3 wherein the step of facilitating exploration of the personal variant sequences comprises:
linking at least one genotype of a personal variant sequence to a four color code representing the relevance of the personal variant sequence;
graphically presenting the linked four color code;
linking at least one genotype of a personal variant sequence to an anatomical feature, and
graphically presenting the linked anatomical feature;
linking at least one genotype of a personal variant sequence to a chromosome or a substructure thereof; and
graphically presenting the linked chromosome or substructure thereof.

9. A method of personal genome sequence data management, the method comprising:
obtaining personal genome sequence data, the personal genome sequence data comprising a plurality of personal variant sequences and a plurality of non-variant sequences;
sorting the obtained personal genome sequence data into a first category of personal genome sequence data comprising the personal variant sequences and a second category of personal genome sequence data comprising the non-variant sequences;
storing the sorted variant sequences in a relational database optimized for fast access to the stored variant sequences;
storing the sorted non-variant sequences in a format configured to maximize compression.
filtering the personal variant sequences with a plurality of genotype-feature correlations to identify a plurality of genetic features;
linking each of the identified plurality of genetic features to a personal variant sequence of the plurality of personal variant sequences;
providing a visual presentation of the identified plurality of genetic features based upon the personal variant sequences;
providing a user interface to facilitate navigation of the personal genome sequence data presented in the visual presentation.

10. The method of claim 9, wherein providing a visual presentation further comprises:
categorizing each of the identified genetic features based upon a relevance of the feature into a plurality of relevance categories, wherein a color code is indicative of the identified relevance category;
presenting the color code of the relevance category for each identified genetic feature in the visual presentation;
presenting a graphical representation of a human body, the graphical representation of the human body comprising graphical representations of a plurality of body systems;
sorting the genetic features by an associated body system;
wherein upon receiving a selection of a body system, presenting the genetic features associated with the selected body system.

11. The method of claim 10, further comprising:
presenting a graphical representation of an organ within the graphical representation of a body system or at least one cell type within the graphical representation of the organ within the graphical representation of a body system;
sorting the genetic features by an associated organ or an associated cell type;
wherein upon receiving a selection of an organ within the graphical representation of a body system or a cell type within the graphical representation of the organ within the graphical representation of a body system, presenting the genetic features associated with the selected organ or the selected cell type.

12. The method of claim 9 further comprising:
presenting a graphical representation of human chromosomes; and
presenting an indication comprising the color code for each identified genetic feature in a location on one of the presented human chromosomes corresponding to a location on that chromosome of the personal variant sequence associated with the identified genetic feature;

13. The method of claim 9, further comprising:
receiving a selection of a genetic feature from the visual presentation;
generating a background information page for the selected genetic feature, the background information page comprising a presentation of at least one informational article regarding the selected genetic feature and a body system associated with the selected genetic feature, in conjunction with the identified relevance category, and the personal variant sequences associated with the selected genetic feature; and
visually presenting the generated background information page.

14. A system for management of personal genome sequence data, the system comprising:
a computer processor operating on an application configured to obtain personal genome sequence data, the personal genome sequence data comprising a plurality of personal variant sequences and a plurality of non-variant sequences, the application sorts the personal genome sequence into a first category comprising the personal variant sequences and a second category comprising the non-variant sequence, and filter the personal variant sequences with a plurality of genotype-feature correlations to identify a plurality of genetic features linked to a personal variant sequence of the plurality;
a first computer readable medium configured to store the personal variant sequences in a relational database for fast access to the stored variant sequences;
a second computer readable medium configured to store the non-variant sequences in a format configured to maximize compression; and
a graphical user interface operating on a graphical display to visually present the plurality of identified genetic features based upon the personal variant sequences and to facilitate navigation of the personal variant sequences.

15. The method of claim 9, further comprising:
linking annotation data to the personal variant sequences;
receiving a search query;
filtering the personal variant sequences by comparing the search query to the annotation data linked to the personal variant sequences;
presenting, on a graphical display, a subset of the personal variant sequences deemed to be the most relevant based upon comparison between the search query to the annotation data.

16. The method of claim 15, further comprising:
calculating a score for each personal variant sequence based upon the annotation data linked to the personal variant sequences; and
providing an indication of the calculated score for each of the personal variant sequences in the presented subset of the personal variant sequences.

17. The method of claim 16, wherein the annotation data comprises the plurality of genetic features.

18. The method of claim 15, wherein the search query is a natural language search query and further comprising parsing key terms from the natural language search query, wherein the personal variant sequences are filtered with the key terms.

19. The method of claim 15, further comprising:
receiving a selection of one of the personal variant sequences of the subset of the personal variant sequences;
presenting, on the graphical display, the annotation data linked to a selected personal variant sequence.

20. The method of claim 15, further comprising:
receiving a selection of one of the personal variant sequences of the subset of the personal variant sequences; and
selecting specific variants, adding personal notes to a variant, adding scientific publications to a variant, or compiling a customized report.
